# EUROPEAN PATENT APPLICATION

(11) **EP 3 239 138 A1**
(43) Date of publication of application: **01.11.2017**
(21) Application number: 16166903.1
(22) Date of filing: 25.04.2016
(51) Int. Cl.: C07D 295/088, A61K 31/4453, A61P 25/28

(54) **HYDROGEN FUMARATE SALT OF 1-[3-[3-(4-CHLOROPHENYL)PROPOXY]PROPYL]PIPERIDINE**

(71) Applicant: SANDOZ AG, 4056 Basel (CH)
(72) Inventor: STEFINOVIC, Marijan, 6250 Kundl (AT); REECE, Hayley, Milton Bridge, EH26 0BE (GB)
(74) Representative: Kluschanzoff, Harald

(57) **Abstract**

The present invention relates to pitolisant hydrogen fumarate and a process for its preparation. The invention also relates to pharmaceutical compositions comprising pitolisant hydrogen fumarate and to the use of pitolisant hydrogen fumarate and of said pharmaceutical compositions as a medicament, in particular for the treatment of narcolepsy with or without cataplexy.

## Description

### FIELD OF THE INVENTION

The present invention relates to pitolisant hydrogen fumarate and a process for its preparation. The invention also relates to pharmaceutical compositions comprising pitolisant hydrogen fumarate and to the use of pitolisant hydrogen fumarate and of said pharmaceutical compositions as a medicament, in particular for the treatment of narcolepsy with or without cataplexy.

### BACKGROUND OF THE INVENTION

Pitolisant, is chemically designated as 1-[3-[3-(4-chlorophenyl)propoxy]propyl]piperidine and can be represented by the following chemical structure according to formula (I):

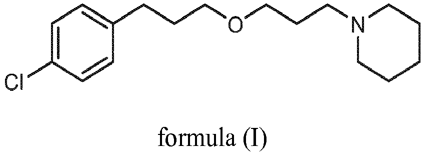

Pitolisant is an antagonist/inverse agonist of the histamine H3 receptor and works by enhancing the histaminergic transmissions in the brain. It is indicated for the treatment of narcolepsy with or without cataplexy.

WO 00/006254 A2 discloses the compound pitolisant and a method for its preparation. Example 117 of the same document provides a concrete procedure for the preparation of pitolisant, which is finally crystallized as the oxalate salt.

According to the later application WO 2006/084833 A1 the use of pitolisant as the free base is limited because of its oily nature. It is further stated that the low solubility of the oxalate salt of pitolisant (0.025 g/ml at 23 °C) also limits its use as a pharmaceutical ingredient. Furthermore, WO 2006/084833 A1 teaches that the low melting points of the hydrogen succinate and hydrogen maleate salts of pitolisant made them less suitable for industrial application. The application provides a hydrochloride salt of pitolisant, wherein the water content of said hydrochloride salt varies from traces of water up to 6 ± 0.5%.

The tendency of a drug substance to absorb water vapor from the environment can affect the pharmaceutical behavior and quality of a drug product containing a hygroscopic substance. Water absorption for example can lead to chemical degradation, trigger changes of the physical form, lead to changes in dissolution behavior and influence powder properties such as flowability, compactability, tableting and compression behavior etc. In addition, variations in water content may also lead to problems related to content uniformity of the final drug product. That is why, non-hygroscopic substances have a constant water content regardless of the surrounding atmosphere and are usually preferred by formulation scientists, as no precautionary measures need to be taken, rendering pharmaceutical processes cheaper and more reliable. Thus, there is a strong need for the provision of a non-hygroscopic salt of pitolisant having a consistent water content.

In addition, chlorides and hydrochloric acid can cause stress corrosion/pitting in stainless steel equipment particularly at low pH. On a large scale, this necessitates use of glass lined vessels or equipment such as centrifuges and pressure filters fabricated from special corrosion resistant alloys (e.g. Hastelloy) *(*P. H. Stahl et al. "Handbook of Pharmaceutical, Chapter 8.1.1 on page 212*)*. Hydrochloric acid is conveniently used as a 36 weight% solution in water. However, as in the case of pitolisant hydrochloride, water interferes with the formation and isolation of the solid crystalline product, necessitating the use anhydrous gas as the chloride source and anhydrous solvents. The latter requirements render a manufacturing process expensive and cumbersome.

Therefore, there is a need for the provision of a salt of pitolisant, in particular a non-hygroscopic salt of pitolisant, which possesses properties allowing for the straight-forward formulation of a pharmaceutical drug product and which can be produced cheaply and easily.

### SUMMARY OF THE INVENTION

The present invention is directed to pitolisant hydrogen fumarate, preferably crystalline pitolisant hydrogen fumarate. In particular, the present invention provides crystalline pitolisant hydrogen fumarate characterized by having a powder X-ray diffractogram comprising reflections at 2-Theta angles of (10.0 ± 0.2)°, (19.6 ± 0.2)° and (24.7 ± 0.2)°, when measured at a temperature in the range of from 20 to 30 °C with Cu-Kalpha_{1,2} radiation having a wavelength of 0.15419 nm.

The invention further relates to a process for the preparation of pitolisant hydrogen fumarate. In addition, the invention relates to a pharmaceutical composition comprising pitolisant hydrogen fumarate. Finally, the present invention relates to the use of pitolisant hydrogen fumarate and of said pharmaceutical composition as a medicament, in particular for the treatment of narcolepsy with or without cataplexy.

### Abbreviations

- PXRD: powder X-ray diffractogram
- DSC: differential scanning calorimetry
- TGA: thermogravimetric analysis
- GMS: gravimetric moisture sorption
- RH: relative humidity

### Definitions

The term "pitolisant" as used herein refers to 1-[3-[3-(4-chlorophenyl)propoxy]propyl] piperidine according to formula (I) disclosed herein above. A process for the preparation is disclosed in WO 00/006254 A2, in particular in example 117, where pitolisant is obtained as intermediate before it is crystallized as the oxalate salt.

The term "pitolisant hydrogen fumarate" as used herein refers to the hydrogen fumarate salt 1-[3-[3-(4-chlorophenyl)propoxy]propyl]piperidine according to formula (II) disclosed hereinafter having a chemical structure comprising about one molecule of pitolisant per molecule of fumaric acid.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-theta. Thus, a reflection that usually appears at 10.0° 2-Theta for example can appear between 9.8° and 10.2° 2-theta, preferably between 9.9 and 10.1° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Standard conditions can also mean a temperature of about 25 °C. Typically, standard conditions can additionally mean a measurement under 20-80% relative humidity, preferably 30-70% relative humidity, more preferably 40-60% relative humidity and most preferably 50% relative humidity.

The term "room temperature" as used herein means a temperature in the range of from 20 to 30 °C.

As used herein, the term "substantially pure" with reference to a particular physical form means that the physical form includes at most 20%, preferably at most 10%, more preferably at most 5%, even more preferably at most 2% and most preferably at most 1% by weight of any other physical form.

The terms "physical form" and "solid form" are used interchangeably herein and refer to any crystalline and/or amorphous phase of a compound.

Crystalline pitolisant hydrogen fumarate may be referred to herein as being characterized by graphical data "as shown in" a figure. Such data include, for example, PXRDs, DSCs, TGAs and GMS isotherms. The person skilled in the art understands that factors such as variations in instrument type, response and variations in sample directionality, sample concentration, sample purity, sample history and sample preparation may lead to variations for such data when presented in graphical form, for example variations relating to the exact peak positions and intensities. However, a comparison of the graphical data in the figures herein with the graphical data generated for an unknown physical form and the confirmation that two sets of graphical data relate to the same crystal form is well within the knowledge of a person skilled in the art.

As used herein, the term "mother liquor" refers to the solution remaining after crystallization of a solid.

The term "non-hygroscopic" as used herein refers to a compound which shows a water uptake of at most 0.5 weight%, based on the weight of the compound, when measured with gravimetric moisture sorption at a relative humidity in the range of from 0 to 90% and a temperature of (25.0 ± 0.1) °C.

The term "anhydrous" as used herein refers to a solid, where no water is coordinated in or accommodated by the crystal structure. However, an anhydrate may still comprise residual water due to surface adsorption, solvent inclusions and/ or absorption in disordered regions.

The term "solvate" as used herein refers to a solid, where one or more organic solvent(s) is/are coordinated in or accommodated by the crystal structure.

The term "hydrate" as used herein refers to a solid, where water is coordinated in or accommodated by the crystal structure.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

### BRIEF DESCRIPTION OF THE FIGURES

**Figure 1****:** illustrates a representative PXRD of crystalline pitolisant hydrogen fumarate of the present invention. The x-axis shows the scattering angle in °2-theta, the y-axis shows the intensity of the scattered X-ray beam in counts of detected photons.
**Figure 2****:** illustrates a representative TGA curve of crystalline pitolisant hydrogen fumarate of the present invention. The x-axis shows the temperature in degree Celsius (°C), the y-axis shows the mass (loss) of the sample in percent (%).

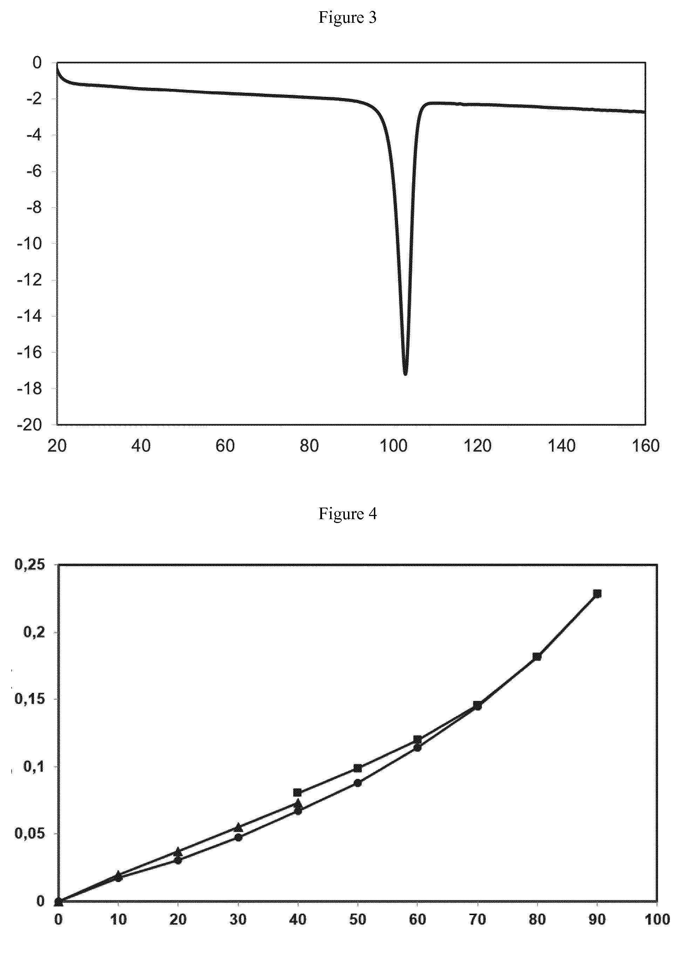

**Figure 3****:** illustrates a representative DSC curve of crystalline pitolisant hydrogen fumarate of the present invention. The x-axis shows the temperature in degree Celsius (°C) (excerpt from about 20 to 160 °C), the y-axis shows the heat flow rate in Watt per gram (W/g) with endothermic peaks going down.
**Figure 4****:** illustrates representative GMS isotherms of crystalline pitolisant hydrogen fumarate of the present invention. The x-axis displays the relative humidity in percent (%) measured at a temperature of (25.0 ± 0.1) °C, the y-axis displays the equilibrium mass change in percent (%). The values are displayed as uncorrected values. The first sorption cycle is labeled as squares, the subsequent desorption cycle as circles and the second sorption cycle as triangles.

### DETAILED DESCRIPTION OF THE INVENTION

The present invention relates to pitolisant hydrogen fumarate, preferably in crystalline form.

Pitolisant hydrogen fumarate can be represented by the following chemical structure according to formula (II):

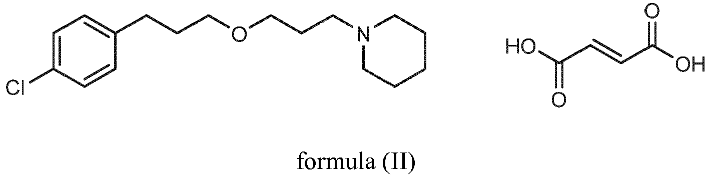

Pitolisant hydrogen fumarate according to the present invention has a molar ratio of pitolisant and fumaric acid typically and preferably in the range of from about 1.0: 0.7 to 1.3, more preferably in the range of from about 1.0: 0.8 to 1.2, even more preferably in the range of from about 1.0: 0.9 to 1.1 and in particular the molar ratio is about 1.0: 1.0.

Crystalline pitolisant hydrogen fumarate of the present invention may be characterized by analytical methods well known in the field of the pharmaceutical industry for characterizing solids. Such methods comprise but are not limited to PXRD, DSC, TGA and GMS. The crystalline pitolisant hydrogen fumarate of the present invention may be characterized by one of the aforementioned analytical methods or by combining two or more of them. In particular, it may be characterized by any one of the following embodiments or by combining two or more of the following embodiments.

Hence, in a first embodiment the present invention relates to crystalline pitolisant hydrogen fumarate characterized by having a PXRD comprising reflections at 2-Theta angles of:
(10.0 ± 0.2)°, (19.6 ± 0.2)° and (24.7 ± 0.2)°, or
(10.0 ± 0.2)°, (17.4 ± 0.2)°, (19.6 ± 0.2)° and (24.7 ± 0.2)°; or
(10.0 ± 0.2)°, (17.4 ± 0.2)°, (19.6 ± 0.2)°, (21.5 ± 0.2)° and (24.7 ± 0.2)°; or
(10.0 ± 0.2)°, (17.4 ± 0.2)°, (19.6 ± 0.2)°, (21.5 ± 0.2)°, (22.6 ± 0.2)° and (24.7 ± 0.2)°; or
(10.0 ± 0.2)°, (13.4 ± 0.2)°, (17.4 ± 0.2)°, (19.6 ± 0.2)°, (21.5 ± 0.2)°, (22.6 ± 0.2)° and (24.7 ± 0.2)°; or
(10.0 ± 0.2)°, (13.4 ± 0.2)°, (16.6 ± 0.2)°, (17.4 ± 0.2)°, (19.6 ± 0.2)°, (21.5 ± 0.2)°, (22.6 ± 0.2)° and (24.7 ± 0.2)°; or
(10.0 ± 0.2)°, (13.4 ± 0.2)°, (16.6 ± 0.2)°, (17.4 ± 0.2)°, (18.8 ± 0.2)°, (19.6 ± 0.2)°, (21.5 ± 0.2)°, (22.6 ± 0.2)° and (24.7 ± 0.2)°; or
(10.0 ± 0.2)°, (13.4 ± 0.2)°, (16.6 ± 0.2)°, (17.4 ± 0.2)°, (18.8 ± 0.2)°, (19.3 ± 0.2)°, (19.6 ± 0.2)°, (21.5 ± 0.2)°, (22.6 ± 0.2)° and (24.7 ± 0.2)°; or
(10.0 ± 0.2)°, (13.4 ± 0.2)°, (16.6 ± 0.2)°, (17.4 ± 0.2)°, (18.8 ± 0.2)°, (19.3 ± 0.2)°, (19.6 ± 0.2)°, (21.5 ± 0.2)°, (22.6 ± 0.2)°, (24.4 ± 0.2)° and (24.7 ± 0.2)°; or
when measured at a temperature in the range of from 20 to 30 °C with Cu-Kalpha_{1,2} radiation having a wavelength of 0.15419 nm.

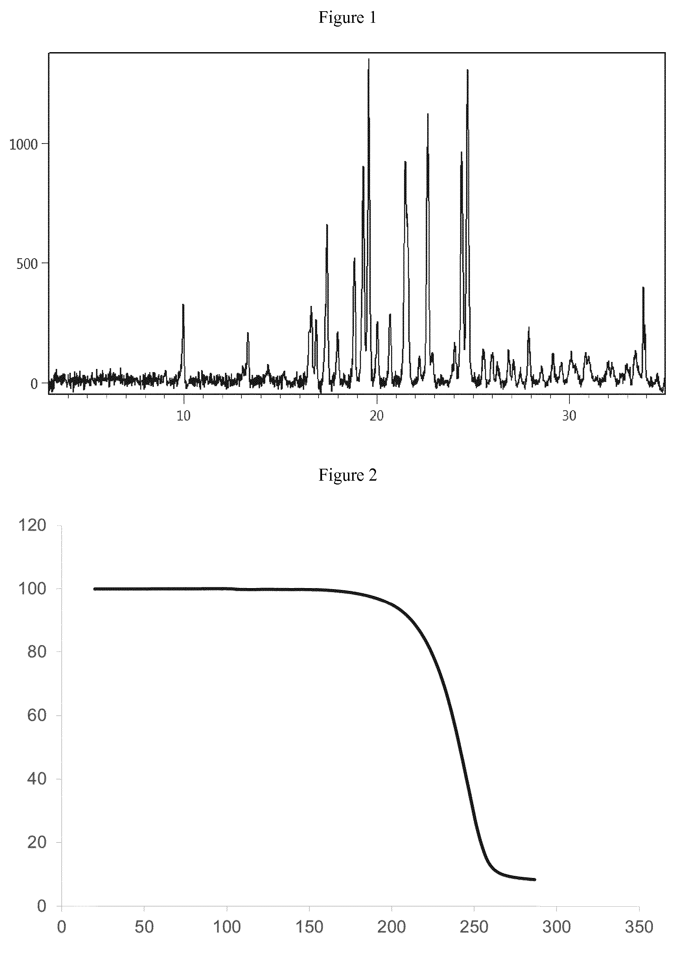

In another embodiment, the present invention relates to crystalline pitolisant hydrogen fumarate characterized by having a PXRD essentially the same as shown in figure 1 of the present invention, when measured at a temperature in the range of from 20 to 30 °C with Cu-Kalpha_{1,2} radiation having a wavelength of 0.15419 nm.

In another embodiment, the present invention relates to crystalline pitolisant hydrogen fumarate characterized by showing a mass loss of not more than 0.5 weight%, preferably not more than 0.4 weight%, more preferably not more than 0.3 weight%, even more preferably not more than 0.2 weight% and most preferably not more than 0.1 weight%, based on the weight of the crystalline pitolisant hydrogen fumarate, when measured with TGA at a temperature in the range of from 25 to 130 °C and a heating rate of 10 °K/min.

In a further preferred embodiment, the present invention relates to crystalline pitolisant hydrogen fumarate characterized by having a melting point onset temperature of (100.0 ± 1.0) °C, for example of 99.5 °C, when measured with DSC at a heating rate of 10 °K/min.

In yet a further preferred embodiment, the present invention relates to crystalline pitolisant hydrogen fumarate characterized by having a melting point peak temperature of (103.0 ± 1.0) °C, for example of 102.8 °C, when measured with DSC at a heating rate of 10 °K/min.

In still another embodiment, the present invention relates to crystalline pitolisant hydrogen fumarate characterized by showing a mass change of not more than 1.0 weight%, preferably not more than 0.5 weight%, more preferably not more than 0.4 weight% and most preferably not more than 0.3 weight%, based on the weight of the crystalline pitolisant hydrogen fumarate, when measured with GMS at a relative humidity in the range of from 0 to 90% and a temperature of (25.0 ± 0.1) °C. Preferably, crystalline pitolisant hydrogen fumarate of the present invention is anhydrous, more preferably it is non-hygroscopic.

Another aspect of the invention concerns a composition comprising the crystalline pitolisant hydrogen fumarate of the present invention, which composition is substantially free of any other physical form of pitolisant and/or of an acid addition salt of pitolisant. For example, a composition comprising crystalline pitolisant hydrogen fumarate of the present invention comprises at most 20 weight%, preferably at most 10 weight%, more preferably at most 5 weight%, even more preferably at most 2 weight% and most preferably at most 1 weight% of any other physical form of pitolisant and/or of an acid addition salt of pitolisant, based on the weight of the composition. Preferably, the any other physical form of the acid addition salt of pitolisant is amorphous pitolisant hydrogen fumarate.

Pitolisant hydrogen fumarate of the present invention possesses solid state properties, which favor its use during pharmaceutical processing and for pharmaceutical compositions. For example, in contrast to the free base, which according to WO 2006/084833 A1 is of oily nature, pitolisant hydrogen fumarate of the present invention is present as a free flowing powder and therefore convenient to handle. In addition, having a melting point onset temperature of about 99.5 °C, pitolisant hydrogen fumarate of the present invention shows a significant higher melting point compared to the hydrogen succinate (melting point of 63.2 °C according to table 7 of WO 2006/084833 A1) and the hydrogen maleate (melting point of 90.9 °C according to table 7 of WO 2006/084833 A1) salts, rendering it more resistant against thermal stress during pharmaceutical processes involving the evolution of heat, such as milling or drying processes.

In addition, the inventors of the present invention surprisingly found that the crystalline hydrogen fumarate salt of the present invention also shows significant improvements over the hydrochloride salt disclosed in WO 2006/084833 A1.

According to WO 2006/084833 A1 the water content of the hydrochloride salt varies from traces of water up to 6 ± 0.5%. This characteristic of the substance is of critical importance as the tendency of a drug substance for absorbing water from the environment can affect the pharmaceutical behavior and quality of a drug product containing such a hygroscopic substance. Water absorption, for example, can lead to chemical degradation, trigger changes of the physical form, lead to changes in dissolution behavior and influence powder properties such as flowability, compactability, tableting and compression behavior etc. In addition, the variable water content of hygroscopic drug substances may also lead to problems related to content uniformity of the final drug product.

Advantageously, the hydrogen fumarate salt of pitolisant of the present invention shows almost no interaction with water vapor at all, but is non-hygroscopic and therefore is characterized by a consistent water content. According to GMS experiments the fumarate salt of the present invention only takes up about 0.2 weight% of water based on the weight of the crystalline pitolisant hydrogen fumarate at a relative humidity in the range of from 0 to 90%. Therefore the non-hygroscopic pitolisant hydrogen fumarate of the present invention is preferred, since no precautionary moisture control measures need to be implemented, making pharmaceutical processing cheap and more reliable.

The pitolisant hydrogen fumarate of the present invention is also advantageous in respect to pitolisant oxalate, because the oxalate salt is not suitable for a long-term use as a medicament due to safety reasons. The regular intake of oxalic acid over a prolonged period of time can cause kidney failure due to the deposit of sparingly soluble calcium oxalate and should therefore be avoided. On the other hand, no nephrotoxicity has been reported for fumaric acid so far *(see* P. H. Stahl et al. "Handbook of Pharmaceutical, Chapter 12, Monographs of oxalic and fumaric acid*).*

In another aspect, the present invention relates to a process for the preparation of crystalline pitolisant hydrogen fumarate of the present invention comprising:
(i) reacting pitolisant with fumaric acid in the presence of a suitable solvent or solvent mixture;
(ii) crystallizing pitolisant hydrogen fumarate from the reaction mixture obtained in step (i);
(iii) optionally separating at least a part of the crystals obtained in step (ii) from their mother liquor;
(iv)optionally washing the isolated crystals obtained in step (iii);
(v) optionally drying the crystals obtained in any one of steps (ii) to (iv);
(vi)optionally slurrying the crystals obtained in any one of steps (iii) to (v) in acetone, water or a mixture thereof;

The pitolisant starting material may be prepared according to the procedure disclosed in WO 00/006254 A2, as described in example 1 of WO 2006/084833 A1 or according to the procedure disclosed in WO 2007/006708 A1.

The starting material provided in step (i) is dissolved in a suitable solvent or solvent mixture. Suitable solvents, which may be applied in the present process are selected from but not limited to the group consisting of ketones, alcohols, nitriles, esters and ethers. A suitable ketone is for example acetone, a suitable alcohol is for example ethanol, a suitable nitrile is for example acetonitrile, a suitable ester is for example ethyl acetate and a suitable ether is for example tetrahydrofuran. Most preferably, the solvent is acetone. The solution may be prepared at a temperature in the range of from about 20 °C to the reflux temperature of the employed solvent(s) or solvent mixture(s), most preferably the solution is prepared at a temperature in the range of from about 20 to 30 °C.

Pitolisant is then reacted with fumaric acid, whereat the pitolisant solution is added to fumaric acid or vice versa. The fumaric acid may be employed as solid or in form of a suspension or solution. In one embodiment, a solution comprising pitolisant and a suspension comprising fumaric acid may be prepared separately from each other and are subsequently combined. The solvents or solvent mixtures used for the preparation of the solution and suspension respectively are preferably the same. The molar ratio of pitolisant to fumaric acid applied typically and preferably is in the range of from about 1.0: 0.7 to 1.3, more preferably in the range of from about 1.0: 0.8 to 1.2, even more preferably in the range of from about 1.0 :0.9 to 1.1 and in particular the molar ratio applied is about 1.0: 1.0. The pitolisant hydrogen fumarate concentration of the final solution obtained in step (i) is preferably in the range of from about 200 to 600 g/L, more preferably from about 300 to 500 g/L and most preferably from about 350 to 450 g/L, for example the concentration is about 400 g/L.

In a subsequent step (ii) the solution obtained from step (i) is subjected to crystallization conditions. Preferably and typically, crystallization occurs spontaneously. The crystallization may also be initiated by subjecting the solution to crystallization conditions comprising any kind of movement, wherein any kind of movement encompasses but is not limited to e.g. agitation, stirring, mixing, shaking, vibration, sonication, scratching and the like. Optionally, seed crystals of pitolisant hydrogen fumarate may be added in order to initiate crystallization and to control crystal growth respectively. The crystallization may also be facilitated by addition of an antisolvent to decrease the solubility of the compound in the solution and to afford the formation of crystals. An antisolvent is a solvent in which the compound has low solubility. Antisolvents are preferably selected from the group consisting of aliphatic and aromatic hydrocarbons and ethers, more preferably pentane, hexane, heptane, octane, toluene, xylene, diethyl ether, t-butyl methyl ether, diisopropyl ether and diisobutyl ether. The crystallization is preferably conducted at room temperature or below, most preferably at room temperature.

Subsequently, at least a part of the crystals are separated from their mother liquor. Preferably, the crystals are separated from their mother liquor by any conventional method such as filtration, centrifugation, solvent evaporation or decantation, more preferably by filtration or centrifugation and most preferably by filtration.

Optionally, in a further step the isolated crystals are washed with a suitable solvent, for example an organic solvent and/or water. Suitable organic solvents comprise but are not limited to ketones, alcohols, nitriles, esters and ethers. A suitable ketone is for example acetone, a suitable alcohol is for example ethanol, a suitable nitrile is for example acetonitrile, a suitable ester is for example ethyl acetate and a suitable ether is for example tetrahydrofuran. Preferably, the isolated crystals are washed with a liquid substantially consisting of the same solvent composition as their mother liquor.

The obtained crystals may optionally be dried. Drying may be performed at a temperature of about 80 °C or less, preferably of about 60 °C or less, more preferably of about 40 °C or less and most preferably drying is performed at about room temperature. Drying may be performed for a period in the range of from about 1 to 72 hours, preferably from about 2 to 48 hours, more preferably from about 4 to 24 hours and most preferably from about 6 to 18 hours. Drying may be performed at ambient pressure and/or under reduced pressure. Preferably, drying is performed at a pressure of about 100 mbar or less, more preferably of about 50 mbar or less and most preferably of about 30 mbar or less, for example a vacuum of about 20 mbar or less.

Optionally, in a further step, pitolisant hydrogen fumarate crystals obtained in any one of the steps (iii) to (v) are subjected to a stir-out period in order to improve crystallinity and polymorphic purity.

Crystalline pitolisant hydrogen fumarate prepared according to the above described procedure may be used as seed crystals in subsequent crystallization procedures for producing crystalline pitolisant hydrogen fumarate.

In another aspect of the present invention, the above described process further comprises the step of converting pitolisant hydrogen fumarate to pitolisant or a salt thereof. The salt of pitolisant may be the hydrochloride salt.

An object of the present invention is thus a process for the preparation of pitolisant or a salt thereof comprising:
(i) reacting pitolisant hydrogen fumarate with a suitable base in the presence of a suitable solvent or solvent mixture;
(ii) a) crystallizing pitolisant free base from the reaction mixture obtained in (i); or
(ii) b) reacting the pitolisant obtained in step (i) with a suitable acid and crystallizing the obtained pitolisant salt
(iii) optionally separating at least a part of the crystals obtained in step (ii) from their mother liquor;
(iv)optionally washing the isolated crystals obtained in step (iii);
(v) optionally drying the crystals obtained in any one of steps (ii) to (iv);

Besides its superior solid state properties, the pitolisant hydrogen fumarate salt of the present invention is also more convenient to manufacture according to the above described procedure compared to the hydrochloride salt of WO 2006/084833 A1.

It is well known by the skilled person that chlorides and hydrochloric acid can cause stress corrosion/pitting in stainless steel equipment particularly at low pH. On a large scale, this necessitates use of glass lined vessels or equipment such as centrifuges and pressure filters being fabricated from special resistant alloys (e.g. Hastelloy) *(*P. H. Stahl et al. "Handbook of Pharmaceutical, Chapter 8.1.1 on page 212*).* Example 2 of WO 2006/084833 A1 also makes use of glass vessels and glass filters for the therein disclosed pitolisant hydrochloride production process.

Hydrochloric acid is conveniently used as the 36 weight% solution in water. However if, as in the case of pitolisant hydrochloride, water interferes with the formation and isolation of the solid crystalline product, it is necessary to use the anhydrous gas as chloride source as well as anhydrous solvents, which renders a manufacturing process expensive and cumbersome. Due to the high water solubility of pitolisant hydrochloride (see table 6 of WO 2006/084833 A1) the presence of water delays or even prevents the crystallization of the salt and leads to a significant yield drop. This is the reason why example 2 of WO 2006/084833 A1 uses a gaseous hydrochloride source and anhydrous ethyl acetate.

An additional advantage of the crystalline pitolisant hydrogen fumarate of the present invention is that chemical impurities are depleted upon crystallization. For example, when introducing pitolisant free base having a chemical purity of 98.1 area% in the above described process the hydrogen fumarate salt of the present invention was recovered with a purity of 99.4 area% as determined by HPLC at 225 nm.

In a further aspect, the present invention relates to a pharmaceutical composition comprising pitolisant hydrogen fumarate and one or more pharmaceutically acceptable excipient(s).

The one or more pharmaceutically acceptable excipient(s) is/are preferably selected from the group consisting of carriers, fillers, diluents, lubricants, glidants, sweeteners, stabilizing agents, solubilizing agents, antioxidants and preservatives, flavouring agents, binders, colorants, osmotic agents, buffers, surfactants, disintegrants, granulating agents, coating materials and combinations thereof.

In a preferred embodiment, the one or more pharmaceutically acceptable excipient(s) is/are selected from the group consisting of diluents, binders, disintegrants, lubricants and glidants. In even a more preferred embodiment, the pharmaceutical composition in addition comprises one or more solubilizing agent(s). Most preferred excipients are selected from the group consisting of microcrystalline cellulose, crospovidone, colloidal silicon dioxide, talc and magnesium stearate.

The pharmaceutical composition of the present invention is preferably an oral solid dosage form, such as a tablet or a capsule. Preferably, the pharmaceutical composition of the present invention is a tablet. In a preferred embodiment, the tablet is film-coated with the commercially available coating material Opadry II white.

Preferably, the pharmaceutical composition comprises an amount of the pitolisant hydrogen fumarate of 4.5 mg, 9 mg, 13.5 mg, 18 mg, 22.5 mg, 27 mg, 31.5 mg or 36 mg calculated as pitolisant free base. Most preferably, the amount of the pitolisant hydrogen fumarate is 4.5 mg or 18 mg calculated as pitolisant free base.

The pharmaceutical composition of the present invention can be prepared by wet or dry processing methods. In certain embodiments the pharmaceutical composition is prepared by wet processing methods, such as, but not limited to, wet granulation methods. Suitable wet granulation methods comprise high-shear granulation or fluid-bed granulation. In another embodiment the pharmaceutical composition is prepared by dry processing methods, such as, but not limited to, direct compression or dry granulation methods. An example of dry granulation is roller compaction. The pharmaceutical composition obtained by dry or wet processing methods may be compressed into tablets, encapsulated or metered into sachets. Preferably, the pharmaceutical composition is compressed into tablets.

In a further aspect, the present invention relates to pitolisant hydrogen fumarate or the pharmaceutical composition comprising the same as described above for use as a medicament.

In yet another aspect, the present invention relates to pitolisant hydrogen fumarate or the pharmaceutical composition comprising the same as described above for use in the treatment of narcolepsy with or without cataplexy.

### EXAMPLES

### Powder X-ray diffraction

Powder X-ray diffraction was carried out on a Siemens D5000 diffractometer, scanning the samples between 3 and 50 °2-theta. For samples of less than 100 mg, 10-20 mg of material was gently compressed onto a glass disc inserted into a PXRD sample holder. For samples of more than 100 mg, ca. 100 mg of material was gently compressed into a plastic PXRD sample holder to ensure a smooth sample surface, just above the level of the sample holder. The sample was then loaded into the diffractometer running in reflection mode and analyzed using the following experimental conditions.

| | |
|---|---|
| Raw Data Origin | Siemens-binary V2 (.RAW) |
| Start Position [°2Th.] | 3.0000 |
| End Position [°2Th.] | 30.000 |
| Step Size [°2Th.] | 0.0200 |
| Scan Step Time [s] | 1 |
| Scan Type | Continuous |
| Offset [°2Th.] | 0.0000 |
| Divergence Slit Type | Fixed |
| Divergence Slit Size [°] | 2.0000 |
| Specimen Length [mm] | various |
| Receiving Slit Size [mm] | 0.2000 |
| Measurement Temperature [°C] | 20.00 |
| Anode Material | Cu |
| K-Alpha1/2 [Å] | 1.5419 |
| K-Beta [Å] | 1.39225 |
| K-A2 / K-A1 Ratio | 0.50000 (nominal) |
| Generator Settings | 40 mA, 40 kV |
| Diffractometer Type | d5000 |
| Goniometer Radius [mm] | 217.50 |
| Incident Beam Monochromator | No |
| Diffracted Beam Monochromator | (Graphite) |
| Spinning | No |

### Differential scanning calorimetry

Differential scanning calorimetry was conducted on a Seiko Exstar DSC6200 instrument. Approximately 5 mg of material was weighed into an aluminium DSC pan and sealed non-hermetically with a pierced aluminium lid. The sample pan was then loaded into a Seiko DSC6200 (equipped with a cooler). The sample and reference were heated to ca. 190 °C at a scan rate of 10 K/min and the resulting heat flow response monitored. The heat flow, which was normalized by sample weight, was plotted versus the measured sample temperature. The data were reported in units of watts/gram ("W/g"). The plot was made with the endothermic peaks pointing down. The endothermic melt peak (melting point) was evaluated for extrapolated onset temperature.

### Thermogravimetric analysis

The TGA instrument used to test the crystalline form was a Seiko Exstar TG/DTA6200. Approximately 5 mg of material was weighed into an open aluminium pan and loaded into a simultaneous thermogravimetric/differential thermal analyser (TG/DTA) and held at room temperature. The sample was then heated at a rate of 10 K/min from 25 °C to ca. 290 °C during which time the change in sample weight was recorded. Nitrogen was used as the purge gas, at a flow rate of 100 cm³/min.

### Gravimetric moisture sorption

Approximately 10-20 mg of sample was placed into a vapour sorption balance pan and loaded into a DVS-1 dynamic vapour sorption balance by Surface Measurement Systems. The sample was subjected to a ramping profile from 40 - 90% relative humidity (RH) at 10% increments, maintaining the sample at each step until a stable weight had been achieved (99.5% step completion). After completion of the sorption cycle, the sample was dried using the same procedure, but all the way down to 0% RH and finally taken back to the starting point of 40% RH. The weight change during the sorption/desorption cycles were plotted.

### Example 1: Preparation of pitolisant hydrogen fumarate

A solution of 1.23 g (4.16 mmol) pitolisant free base (prepared according to the procedure disclosed in WO 2007/006708 A1) in 1 mL acetone was slowly added to a suspension of 0.48 g (4.14 mmol) fumaric acid in 1.5 mL acetone at room temperature. During addition, the solution became clear and then after complete addition, crystallization was observed. After 0.5 h of stirring additional 5 mL acetone were added and the suspension was further stirred at room temperature for 2 hours. Thereafter, the temperature was cycled between 5 and 40 °C for 18 hours before 6 mL n-heptane were slowly added over a period of 4 hours. The solid was then separated by centrifuge filtration and dried under vacuum at room temperature for about 60 hours. Finally, the dried solid was suspended in acetone at a concentration of 100 mg/mL, slurried for 3 days at room temperature, filtered and dried under vacuum at room temperature for 16 hours to obtain crystalline pitolisant hydrogen fumarate.

### Example 2: Powder X-ray diffraction

A representative list of reflections including relative intensities of crystalline pitolisant hydrogen fumarate of the present invention is provided in table 2.

**Table 2: Positions and relative intensities of pitolisant hydrogen fumarate reflections having a relative intensity of 10% or more**

| **Position of reflection [± 0.2° 2-Theta]** | **Relative intensity [%]** | **Position of reflection [± 0.2° 2-Theta]** | **Relative intensity [%]** |
|---|---|---|---|
| 10.0 | 22 | 20.1 | 19 |
| 13.4 | 14 | 20.7 | 21 |
| 16.6 | 19 | 21.5 | 68 |
| 16.9 | 18 | 22.6 | 83 |
| 17.4 | 48 | 24.1 | 11 |
| 18.0 | 16 | 24.4 | 65 |
| 18.8 | 38 | 24.7 | 95 |
| 19.3 | 68 | 27.9 | 16 |
| 19.6 | 100 | 33.8 | 31 |

### Example 3: Thermoanalytical investigations

According to TGA crystalline pitolisant hydrogen fumarate showed a mass loss of only 0.1 weight% from the beginning of the measurement up to a temperature of about 130 °C. DSC revealed that the material shows a single melting endotherm with an onset temperature of 99.5 °C and a peak temperature of 102.8 °C.

### Example 4: Gravimetric moisture sorption

Gravimetric moisture sorption revealed that pitolisant hydrogen fumarate practically shows no interaction with water vapor in the range of from 0 to 90% RH. The mass change in the sorptive mode from 0 to 90% RH was with 0.2 weight% negligible. According to the GMS experiment crystalline pitolisant hydrogen fumarate is non-hygroscopic. In addition, no significant hysteresis can be observed, which indicates that pitolisant hydrogen fumarate underwent no significant structural changes. The PXRD of the sample after the GMS experiment was unchanged.

### Example 5: Stability testing

Crystalline pitolisant hydrogen fumarate (20 mg, 99.4 area%) was exposed to three specific conditions. After 7 and 14 days the samples were analyzed by HPLC and PXRD respectively. The samples showed good chemical and physical stability as summarized in table 3.

**Table 3: Results of stability testing**

| **Conditions** | **Purity after 7 days** | **Purity after 14 days** | **PXRD after 14 days** |
|---|---|---|---|
| 40 °C/75% RH (climate chamber) | 99.0 area% | 99.0 area% | unchanged |
| 80 °C (drying oven) | 98.9 area% | 98.9 area% | unchanged |
| RT, ambient light exposure | 98.2 area% | 98.5 area% | unchanged |

### Example 6: Salt disproportionation study

Crystalline pitolisant hydrogen fumarate (40 mg) was weighed into three vials to which a small amount of water was added to form a slurry. The slurries were then left at room temperature for 1,24 and 48 hours respectively. The pH of the supernatant was analyzed post-separation at each time point and the solid was analyzed by PXRD. Pitolisant hydrogen fumarate salt remained stable and did not disproportionate. The results are summarized in table 4.

**Table 4: Results of salt disproportionation study**

| **Time Period** | **pH** | **Sample Observations** | **PXRD** |
|---|---|---|---|
| 1 h | 4.40 | white slurry | unchanged, no counterion traceable |
| 24 h | 3.31 | white slurry | unchanged, no counterion traceable |
| 48 h | 3.33 | white slurry | unchanged, no counterion traceable |

## Claims

1. Pitolisant hydrogen fumarate.

2. Pitolisant hydrogen fumarate according to claim 1 in crystalline form.

3. Pitolisant hydrogen fumarate according to claim 1 or 2, **characterized by** having a powder X-ray diffractogram comprising reflections at 2-Theta angles of (10.0 ± 0.2)°, (19.6 ± 0.2)° and (24.7 ± 0.2)°, when measured at a temperature in the range of from 20 to 30 °C with Cu-Kalpha_{1,2} radiation having a wavelength of 0.15419 nm.

4. Pitolisant hydrogen fumarate according to claim 3, wherein the powder X-ray diffractogram comprises an additional reflection at one or more 2-Theta angle(s) selected from the group consisting of (17.4 ± 0.2)°, (21.5 ± 0.2)° and (22.6 ± 0.1)°.

5. Pitolisant hydrogen fumarate according to any one of the preceding claims, **characterized by** showing a mass loss of less than 0.5 weight%, based on the weight of pitolisant hydrogen fumarate, when measured with thermogravimetric analysis at a temperature in the range of from 25 to 130 °C and a heating rate of 10 K/min.

6. Pitolisant hydrogen fumarate according to any one of the preceding claims, **characterized by** having a melting point onset temperature of (100 ± 1) °C, when measured with differential scanning calorimetry at a heating rate of 10 K/min.

7. Pitolisant hydrogen fumarate according to any one of the preceding claims, **characterized by** showing a mass change of at most 0.3 weight%, based on the weight of pitolisant hydrogen fumarate, when measured with gravimetric moisture sorption at a relative humidity in the range of from 0 to 90% and a temperature of (25.0 ± 0.1) °C.

8. A process for the preparation of pitolisant hydrogen fumarate according to any one of claims 1 to 7, comprising the steps of:
(i) reacting pitolisant free base with fumaric acid in the presence of a suitable solvent or solvent mixture;
(ii) crystallizing pitolisant hydrogen fumarate from the reaction mixture obtained in (i);

9. The process according to claim 8, wherein the solvent or solvent mixture is selected from the group consisting of ketones, alcohols, nitriles, esters, ethers and mixtures thereof.

10. The process according to claim 9, wherein the ketone is acetone, the alcohol is ethanol, the nitrile is acetonitrile, the ester is ethyl acetate and the ether is tetrahydrofuran.

11. The process according to claims 8 to 10 further comprising the step of converting pitolisant hydrogen fumarate to pitolisant or a pharmaceutically acceptable salt thereof and isolating it.

12. A pharmaceutical composition comprising pitolisant hydrogen fumarate according to claims 1 to 7 and one or more pharmaceutically acceptable excipient(s).

13. Pitolisant hydrogen fumarate according to claims 1 to 7 or the pharmaceutical composition of claim 12 for use as a medicament.

14. Pitolisant hydrogen fumarate according to claims 1 to 7 or the pharmaceutical composition of claim 12 for use in the treatment of narcolepsy with or without catalepsy.
